# EUROPÄISCHE PATENTSCHRIFT

(11) **EP 1 669 337 B1**
(45) Veröffentlichungstag und Bekanntmachung des Hinweises auf die Patenterteilung: **13.06.2007**
(21) Anmeldenummer: 05109524.8
(22) Anmeldetag: 13.10.2005
(51) Int. Cl.: C07C 29/141, C07C 29/16, C07C 45/50, B01J 31/16

(54) **Verfahren zur Herstellung von Alkoholen aus Olefinen durch Hydroformylierung und Hydrierung**
Method for the preparation of alcohols from alkenes by means of hydroformylation and hydrogenation
Procédé pour la fabrication d'alcools à partir d'oléfines par hydroformylation et hydrogenation

(30) Priorität: 09.12.2004 DE 102004059292
(43) Veröffentlichungstag der Anmeldung: 14.06.2006
(73) Patentinhaber: Oxeno Olefinchemie GmbH, 45772 Marl (DE)
(72) Erfinder: Lüken, Hans-Gerd, 45770, Marl (DE); Kaizik, Alfred, 45772, Marl (DE); Büschken, Wilfried, 45721, Haltern am See (DE); Stenert, Andreas, 48249, Dülmen (DE)
(74) Vertreter: Hirsch, Hans-Ludwig

(56) Entgegenhaltungen:
- DE-A1- 10 227 995
- US-A- 4 447 661
- US-B1- 6 239 318

## Beschreibung

Die vorliegende Erfindung betrifft ein Verfahren zur Herstellung von Alkoholen durch Hydroformylierung von Olefinen oder Olefmgemischen in Gegenwart eines Kobaltkatalysators, Abtrennen des Katalysators und anschließende Hydrierung.

Höhere Alkohole, insbesondere solche mit 7 bis 25 Kohlenstoffatomen, können bekanntlich durch katalytische Hydroformylierung (auch als Oxo-Reaktion bezeichnet) der um ein Kohlenstoffatom ärmeren Olefine und durch anschließende Hydrierung der gebildeten Aldehyde hergestellt werden. Die Alkohole können als Lösemittel oder als Vorstufe für Detergenzien oder Weichmacher genutzt werden.

Verfahren zur Hydroformylierung von Olefinen sind in der Literatur in großer Zahl bekannt. In EP 0 562 451 und EP 0 646 563 wird die Hydroformylierung von 1- und 2-Buten enthaltenden Mischungen beschrieben, wobei in der ersten Stufe das 1-Buten in einer heterogenen Reaktion, also in einem Mehrphasensystem, gegebenenfalls unter Zusatz eines Phasentransferreagenz oder Lösungsvermittlers umgesetzt wird und in der zweiten Stufe ein homogen gelöster Katalysator zum Einsatz kommt. Gemäß EP 0 562 451 werden in beiden Stufen RhodiumKatalysatoren eingesetzt, während nach EP 0 646 563 in der ersten Stufe Rhodium- und in der zweiten Stufe Kobaltkatalysatoren verwendet werden. Nach EP 0 562 451 wird das in der ersten Stufe nicht umgesetzte Olefm, vorwiegend 2-Buten, in einer zweiten Stufe in homogener Phase und in Gegenwart von Rhodium als Katalysator hydroformyliert. In EP 0 646 563 wird diese Arbeitsweise dahingehend präzisiert, dass die in der ersten Stufe nicht umgesetzten Olefine gasförmig, zusammen mit Kohlenmonoxid, Wasserstoff und durch Hydrierung entstandenem Butan, den Reaktor verlassen, d. h. es wird eine Zwischenabtrennung der Olefine durchgeführt. Das abgetrennte Gas wird, gegebenenfalls nach Komprimierung, in die zweite Hydroformylierungsstufe geführt.

In GB 1 387 657 wird eine zweistufige Hydroformylierung beschrieben, bei der das Reaktionsprodukt der ersten Stufe gasförmig ausgetragen wird und nach Auskondensation der Aldehyde bzw. Alkohole das Abgas der ersten Stufe, das nicht umgesetzte Olefme enthält, zum einen Teil in die erste Stufe zurückgeführt und zum anderen Teil in einen zweiten Reaktor geleitet wird.

Eine weitere Variante einer zweistufigen Hydroformylierung ist in DE 32 32 557 beschrieben. In der ersten Stufe werden die Olefine unter Verwendung eines Kobaltkatalysators mit Umsätzen von 50 bis 90 % hydroformyliert, der Kobaltkatalysator vom Reaktionsgemisch abgetrennt und die gebildeten Aldehyde zusammen mit den nicht umgesetzten Olefinen in eine zweite Hydroformylierungsstufe eingebracht. Der hier eingesetzte ligandmodifizierte Kobaltkatalysator bewirkt nicht nur die Hydroformylierung der Olefine, sondern gleichzeitig eine Hydrierung der Aldehyde zu den Alkoholen.

In DE 100 34 360 wird ein Verfahren zur mehrstufigen Kobalt- oder Rhodium-katalysierten Hydroformylierung von Olefinen mit 6 bis 24 Kohlenstoffatomen zu Alkoholen und/oder Aldehyden beschrieben, wobei die Olefine
a) in einem Hydroformylierungsschritt bis zu einem Umsatz von 20 bis 98 % hydroformyliert werden,
b) der Katalysator aus dem so erhaltenen flüssigen Reaktoraustrag entfernt wird,
c) das so erhaltene flüssige Hydroformylierungsgemisch in eine Leichtsiederfraktion, enthaltend Olefine und Paraffine und eine Sumpffraktion, enthaltend Aldehyde und/oder Alkohole getrennt wird, die in der Leichtsiederfraktion enthaltenden Olefine in weiteren Verfahrensstufen, umfassend die Verfahrensschritte a, b und c umgesetzt werden,
und die Sumpffraktionen der Verfahrensschritte c) aller Verfahrensstufen vereinigt werden.

Bevorzugt wird dieses Verfahren so ausgeübt, dass der flüssige Reaktoraustrag der Hydroformylierungsschritte a) eine homogene Flüssigphase ist. Die Kobalt- oder RhodiumKatalysatoren werden bevorzugt so eingesetzt, dass sie homogen im flüssigen Reaktoraustrag der Hydroformylierungsschritte a) gelöst sind.

In DE 198 42 368 A1 wird ein Verfahren zur Herstellung von höheren Oxo-Alkoholen aus Gemischen isomerer Olefine mit 5 bis 24 Kohlenstoffatomen durch zweistufige Hydroformylierung in Gegenwart eines Kobalt- oder Rhodium-Katalysators bei erhöhter Temperatur und erhöhtem Druck beschrieben, bei dem man das Reaktionsgemisch der ersten Hydroformylierungsstufe selektiv hydriert, das Hydrierungsgemisch in einer Destillation in rohen Alkohol und überwiegend aus Olefmen bestehende Leichtsieder trennt, diese in die zweite Hydroformylierungsstufe führt, das Reaktionsgemisch der zweiten Hydroformylierungsstufe wiederum selektiv hydriert, das Hydriergemischgemisch in einer Destillation in rohen Alkohol und Leichtsieder trennt, den rohen Alkohol durch Destillation auf reinen Alkohol aufarbeitet und zumindest einen Teil der Leichtsieder zur Ausschleusung gesättigter Kohlenwasserstoffe abzieht.

Die in den nach der Hydroformylierung in Gegenwart eines Kobalt-Katalysators erhaltenen organischen Phasen verbleibende Restmengen an Kobalt-Katalysator liegen in der Regel bei weniger als 5 ppm Kobalt (gerechnet als Metall). Bereits diese geringen Kobaltrestmengen können mit zunehmender Betriebszeit negative Auswirkungen sowohl auf die Hydrierung als auch auf die destillative Aufarbeitung haben.

Es wurde festgestellt, dass die eingesetzten Hydrierkatalysatoren mit der Betriebszeit durch die Kobaltrestgehalte in der organischen Phase desaktiviert werden. Es wurden insbesondere bei längerem Betrieb Kobaltablagerung auf der Katalysatoroberfläche beobachtet.

Neben der Katalysatordesaktivierung wird durch die Kobaltablagerungen auch die Hydrodynamik und der Stoff- und/oder Wärmetransport im Hydrierreaktor beeinträchtigt.

In EP 1 057 803 wird ein zweistufiges Verfahren zur Herstellung von Alkoholen aus Olefmen oder Olefingemischen offengelegt. Dabei wird in der ersten Reaktionsstufe das Einsatzolefin in Gegenwart eines Kobaltkatalysators zu 50 bis 90 % hydroformyliert. Nach der Abtrennung des Katalysators werden vom Reaktionsaustrag die nicht umgesetzten Olefme destillativ abgetrennt und die abgetrennten Olefme im zweiten Hydroformylierungsreaktor umgesetzt. Die Hydroformylierungsprodukte aus beiden Stufen können zu den entsprechenden Alkoholen hydriert werden. In beiden Reaktionsstufen wird als Katalysator Co₂(CO)₈ oder HCo(CO)₄ eingesetzt, der außerhalb der Hydroformylierungsreaktoren erzeugt wird. Aus dem Reaktionsgemisch der Hydroformylierung wird vor Weiterverarbeitung durch Extraktion mit einer Base der Kobaltkatalysator entfernt.

In den meisten aus der Literatur bekannten, kobaltkatalysierten Hydroformylierungsverfahren wird der Kobaltkatalysator (HCo(CO)₄ oder Co₂(CO)₈) nach dem Hydroformylierungsschritt oxidativ zerstört. Dies erfolgt in der Regel durch Umsetzen des Hydroformylierungsaustrags mit Luft in Gegenwart einer wässrigen Phase, wobei die so erzeugten Kobalt-II-Salze in die wässrige Phase extrahiert werden. Die Abtrennung der wässrigen Phase erfolgt z. B. durch Dekantieren in einem Phasentrennbehälter oder in anderen dafür geeigneten Einrichtungen. Die organische Phase wird nach Abtrennung von der wässrigen Phase einer katalytischen Hydrierung zugeführt.

In DE 102 27 995.0 wird die Konzentration an Kobaltverbindungen (berechnet als metallisches Kobalt) in den Hydroformylierungsgemischen durch Extraktion mit Wasser auf Werte unter 0,5 Massen-ppm gesenkt. Dadurch kann die Standzeit des Hydrierkatalysators bezüglich ausreichender Hydrierleistung auf circa 2 bis 3 Jahre gebracht werden. Es bleibt jedoch weiterhin der Nachteil bestehen, dass bei der Hydrierung des extrahierten Hydroformylierungsgemisches die erste Katalysatorschicht durch abgelagertes metallisches Kobalt und gegebenenfalls andere Stoffe verklebt wird. Dadurch bedingt baut sich im Reaktor ein zunehmender Differenzdruck auf und die Strömung der zu hydrierenden Flüssigkeit wird in den folgenden Katalysatorschichten ungleichmäßig, sodass die Hydrierleistung zurückgeht. In gewissen zeitlichen Abständen muss die Hydrierung abgestellt werden, um die erste Katalysatorschicht aufzulockern oder durch frischen Katalysator zu ersetzen.

Um solche betrieblichen Unterbrechungen mit den dadurch bedingten Produktionsausfällen und Kosten zu vermeiden, bestand die Aufgabe der Erfindung darin, das Verfahren derart zu verbessern, dass solche Unterbrechungen nur noch selten vorgenommen werden müssen.

Es wurde nun gefunden, dass die Zunahme des Differenzdruckes durch Ablagerungen in der ersten Katalysatorschicht eines Hydrierreaktors reduziert werden kann, wenn das zu hydrierende Gemisch vor dem Eintritt in den Hydrierreaktor ein Adsorptionsbett, in dem Kobaltverbindungen zurückgehalten werden, durchfließt.

Gegenstand der vorliegenden Erfindung ist ein Verfahren zur Herstellung von aliphatischen Alkoholen mit 7 bis 25 Kohlenstoffatomen, bei dem zumindest einmal die Verfahrensschritte
a) Kobalt-katalysierte Hydroformylierung von Olefinen mit 6 bis 24 Kohlenstoffatomen,
b) Behandlung eines Hydroformylierungsgemisches mit sauerstoffhaltigen Gasen in Gegenwart von sauren, wässrigen Kobalt(II)salzlösungen,
c) Trennung eines gemäß Schritt b) erhaltenen Gemisches in eine Kobaltsalze enthaltende wässrige und eine die aliphatischen Aldehyde enthaltende organische Phase und
d) Hydrierung einer aldehydhaltigen organischen Phase durchgeführt werden, welches dadurch gekennzeichnet ist, dass die Aldehyd-haltige organische Phase vor der Hydrierung d) zur Abtrennung von Kobaltverbindungen zumindest teilweise in einem Schritt e) mit einem Adsorptionsmittel behandelt wird.

Das erfindungsgemäße Verfahren hat den Vorteil, dass durch den Einsatz von Adsorbern der in den organischen Phasen vorhandene Anteil an Kobalt-Katalysator vollständig bzw. nahezu vollständig entfernt werden kann. Auf diese Weise kann verhindert werden, dass sich im Hydrierungsreaktor durch Ablagerung von Katalysator-Resten ein zunehmender Differenzdruck aufbaut und eine ungleichmäßige Strömung in den Katalysatorschichten erfolgt. Durch das erfindungsgemäße Verfahren können deshalb die Beschaffungskosten für Ersatzbefüllungen an Katalysator auf das notwendige Maß begrenzt werden. Zudem werden die Rüstkosten für den häufigen Einbau des Katalysators eingespart. Des weiteren werden durch den erfmdungsgemäßen Einsatz des Adsorbers Produktionsausfälle durch Betriebsunterbrechungen vermieden. Da auch als Hydrierkatalysator nicht mehr geeigneter gebrauchter Hydrierkatalysator als Adsorbermaterial geeignet ist, können durch Nutzung der gebrauchten Hydrierkatalysatoren als Adsorbermaterialien die Betriebskosten weiter gesenkt werden.

Das erfindungsgemäße Verfahren wird nachfolgend beispielhaft beschrieben, ohne dass die Erfindung auf die beispielhaften Ausführungsformen beschränkt sein soll. Sind nachfolgend Bereiche, allgemeine Formeln oder Verbindungsklassen angegeben, so sollen diese nicht nur die entsprechenden Bereiche oder Gruppen von Verbindungen offenbaren, die explizit erwähnt sind, sondern auch alle Teilbereiche und Teilgruppen von Verbindungen, die durch Weglassen von einzelnen Werten (Bereichen) oder Verbindungen erhalten werden können.

Das erfindungsgemäße Verfahren zur Herstellung von aliphatischen Alkoholen mit 7 bis 25 Kohlenstoffatomen bei dem zumindest einmal die Verfahrensschritte
a) Kobalt-katalysierte Hydroformylierung von Olefinen mit 6 bis 24 Kohlenstoffatomen,
b) Behandlung eines Hydroformylierungsgemisches mit Sauerstoff-haltigen Gasen in Gegenwart von sauren, wässrigen Kobalt(II)salzlösungen,
c) Trennung eines gemäß Schritt b) erhaltenen Gemisches in eine Kobaltsalze enthaltende wässrige und eine die aliphatischen Aldehyde enthaltende organische Phase und
d) Hydrierung einer Aldehyd-haltigen organischen Phase durchgeführt werden, zeichnet sich dadurch aus, dass die Aldehyd-haltige organische Phase vor der Hydrierung d) zur Abtrennung von Kobaltverbindungen zumindest teilweise in einem Schritt e) mit einem Adsorptionsmittel behandelt wird.

Im erfindungsgemäßen Verfahren können alle Adsorptionsmittel eingesetzt werden, die in der Lage sind aus einer organischen Phase, die Aldehyde und gegebenenfalls Wasser enthält, Kobaltverbindungen abzutrennen, mit der Einschränkung, dass die Adsorptionsmittel unter Adsorptionsbedingungen (nahezu) nicht die Umsetzung von Wertprodukten (Aldehyden, Alkoholen und deren Formiaten) zu Nebenprodukten katalysieren dürfen.

Die Adsorptionsmittel sind vorzugsweise feste, in der Aldehydphase und vorzugsweise auch in Wasser unlösliche Stoffe. Die eingesetzten Adsorptionsmittel können z. B. organische Harze mit funktionellen Gruppen sein, die Kobalt und gegebenenfalls andere Metalle binden können. Die Bindung kann durch Ionenaustausch, Chelatisierung, andere Reaktionen oder sonstige Koordinierung erfolgen.

Eine andere Gruppe von als Adsorptionsmittel im erfmdungsgemäßen Verfahren einsetzbaren Stoffen sind anorganische Feststoffe, die vorzugsweise porös sind und somit eine große Oberfläche aufweisen. Solche anorganischen Feststoffe können beispielsweise Aktivkohle oder Titandioxid, Zirkondioxid, Aluminiumoxid, Siliziumdioxid oder Alumosilikate in ihren verschiedenen Modifikationen sein.

Als Adsorptionsmittel wird vorzugsweise ein Aluminiumoxid (Al₂O₃), Siliziumoxid (SiO₂), Alumosilikate oder Aktivkohle oder ein zumindest einen dieser Stoffe aufweisendes Material eingesetzt. Das Aluminiumoxid, die Alumosilikate und das Siliziumoxid kann in allen bekannten Modifikationen vorliegen.

Es kann vorteilhaft sein, wenn als Adsorptionsmittel gebrauchte oder neue (Hydrier)Katalysatoren, besonders bevorzugt gebrauchte Hydrierkatalysatoren eingesetzt werden. Auf diese Weise können die Katalysatoren, die wegen mangelnder Aktivität für die Hydrierung, z. B. in Schritt d), nicht mehr geeignet sind, zur Entfernung von Kobaltanteilen aus der aldehydhaltigen Phase eingesetzt werden, so dass eine längere Standzeit für die Katalysatoren erreicht wird. Als Adsorptionsmittel werden vorzugsweise Hydrierkatalysatoren eingesetzt, die Aluminiumoxid (Al₂O₃), Siliziumoxid (SiO₂), Alumosilikate oder Aktivkohle als Trägermaterialien aufweisen.

Besonders bevorzugt werden als Adsorbermaterialien solche eingesetzt, die große Oberfläche aufweisen. Bevorzugte Adsorbermaterialien weisen BET-Oberflächen von 80 bis 300 m²/g, vorzugsweise 120 bis 280 m²/g und besonders bevorzugt 180 bis 250 m²/g auf (Bestimmt nach dem BET-Verfahren durch Stickstoff-Adsorption gemäß DIN 66 131). Ganz besonders bevorzugt werden als Adsorbermaterialien Aluminiumoxide mit einer BET-Oberfläche von 80 bis 300 m²/g, vorzugsweise 120 bis 280 m²/g und besonders bevorzugt 180 bis 250 m²/g eingesetzt.

Das Adsorptionsmittel wird vorteilhaft in einer Form eingesetzt, in der es einen geringen Strömungswiderstand bietet, z. B. in Form von Granalien, Pellets oder Formkörper, wie Tabletten, Zylinder, Strangextrudaten oder Ringen.

Für die Adsorption der Kobaltverbindungen aus der flüssigen organischen Phase kann/können in der vorliegenden Erfindung ein oder mehrere Adsorptionsmittel eingesetzt werden. Beispielsweise kann ein Adsorptionsmittel in unterschiedlichen Formen oder Größen eingesetzt werden. Es ist auch möglich Adsorptionsmittel aus unterschiedlichen Materialien (unterschiedliche chemische Zusammensetzung oder unterschiedliche Modifikation) einzusetzen.

Bei Einsatz von mindestens zwei unterschiedlichen Adsorptionsmitteln können diese gemischt oder geschichtet im Adsorber vorliegen. Bei Verwendung mehrerer Adsorber können die einzelnen Adsorber gleiche oder unterschiedliche Adsorptionsmittel oder deren Gemische enthalten.

Weiterhin kann das Adsorptionsmittel Inertmaterialien, wie beispielsweise Glaskugeln aufweisen. Durch diese Glaskugeln kann das Adsorptionsmittel verdünnt werden. Dies hat den Vorteil, dass die Gefahr des Verklebens der Partikel des Adsorptionsmittel verringert wird, die Bildung von lokalen thermischen Überhitzungen ("hot spots") vermieden wird und gegebenenfalls der Differenzdruck im Adsorptionsbehälter verringert werden kann. Optional können die Absorptionsmittel 0,01 bis 2 Massen-% basische Stoffe, wie beispielsweise Verbindungen der Alkalimetalle, der Erdalkalimetalle oder Zink erhalten.

Der Schritt e) (Die Adsorption) kann diskontinuierlich oder kontinuierlich z. B. in Rührkesseln oder Strömungsreaktoren durchgeführt werden. Im erfindungsgemäßen Verfahren wird die Adsorption bevorzugt kontinuierlich in Strömungsreaktoren (Strömungsrohren) an stückigen Adsorptionsmittel durchgeführt.

Der Schritt e) wird vorzugsweise bei einer Temperatur im Bereich von 5 bis 250 °C, bevorzugt von 10 bis 100 °C, besonders bevorzugt von 15 bis 75 °C und besonders bevorzugt von 20 bis 50 °C durchgeführt. Der Druck, bei dem der Schritt e) im erfindungsgemäßen Verfahren durchgeführt wird beträgt vorzugsweise von 0,1 bis 20 MPa, bevorzugt von 0,1 bis 5 MPa und besonders bevorzugt bei 0,2 bis 0,5 MPa.

Als Adsorber, also einer Apparatur, in welcher der Schritt e) durchgeführt werden kann, können übliche geschlossene technische Gefäße mit Zu- und Ablauf an entgegengesetzt gelegenen Stellen des Gefäßes verwandt werden. Im erfmdungsgemäßen Verfahren werden insbesondere Rohrreaktoren als Adsorber eingesetzt. Diese können in Schlaufenfahrweise oder bevorzugt im geraden Durchgang betrieben werden. Senkrecht stehende Rohrreaktoren werden von unten nach oben oder umgehrt durchströmt. Im erfindungsgemäßen Verfahren wird die Strömung von oben nach unten bevorzugt.

Die Leerrohrgeschwindigkeit im Adsorber liegt vorzugsweise im Bereich von 10 m/h bis 300 m/h, bevorzugt im Bereich von 50 m/h bis 150 m/h und besonders bevorzugt im Bereich von 75 bis 120 m/h. Die spezifische Belastung (LHSV) des Adsorptionsmittel (Volumen organischer Phase in Liter je Liter Adsorptionsmittel je Stunde) ist abhängig vom eingesetzten Adsorptionsmittel und der Kobaltkonzentration im Zulauf. Beispielsweise liegt die LHSV bei einer Kobaltkonzentration von 50 bis 150 Massen-ppb und γ-Aluminiumoxid als Adsorptionsmittel bevorzugt in einem Bereich von 40 h⁻¹ bis 80 h⁻¹.

Die erfmdungsgemäße Adsorption gemäß Schritt e) kann ohne Zusatz oder mit Zusatz von Wasserstoff durchgeführt werden. Insbesondere kann der Schritt e) innerhalb oder außerhalb eines Reaktors durchgeführt werden, in dem eine Hydrierung gemäß Schritt d) durchgeführt wird. Erfolgt die Adsorption in Gegenwart von Wasserstoff ist der Adsorber vorzugsweise im Hydrierreaktor integriert. Dies kann z. B. dadurch erreicht werden, dass der Hydrierreaktor zwei Zonen aufweist, eine Zone, die mit dem Hydrierkatalysator ausgerüstet ist und eine Zone, die mit dem Absorptionsmittel ausgestattet ist, wobei die Zone mit dem Absorptionsmittel zwischen Zulauf zum Reaktor und der Zone mit dem Hydrierkatalysator angeordnet ist. Der Vorteil dabei ist, dass ein Apparat eingespart wird. Man hat jedoch den Nachteil, dass bei einem Wechsel des Adsorptionsmittel die Hydrierung unterbrochen werden muss. Zweckmäßig ist es daher, die Adsorption außerhalb des Hydrierreaktors vorzunehmen.

Die Adsorption der Kobaltverbindungen kann in einem oder mehreren Adsorber(n) durchgeführt werden. Bei der Verwendung nur eines Adsorbers außerhalb des Hydrierreaktors hat man bei Wechsel des Adsorptionsmittel die Wahl zwischen Abstellung der Hydrierung und Zuführung einer höheren Kobaltmenge auf den Hydrierkatalysator. Beides ist nachteilig. Daher wird im erfmdungsgemäßen Verfahren der Schritt e) vorzugsweise unter Verwendung von mindestens zwei Adsorbern durchgeführt, weil dann auch bei Außerbetriebnahme eines Adsorbers wegen Wechsel des Adsorptionsmittels oder dessen Regenerierung die Hydrierung ohne zusätzliche Belastung des Katalysators mit Kobaltverbindungen aufrechterhalten werden kann.

Sind im erfindungsgemäßen Verfahren für die Durchführung des Schrittes e) mehr als ein Adsorber vorhanden, so können diese unterschiedlich miteinander verschaltet sein, nämlich in Reihenschaltung, Parallelschaltung oder beim Vorhandensein von mehr als zwei Adsorbern in einer Kombination beider Arten. Wenn alle Adsorber in Reihe geschaltet sind, muss jeder Adsorber mit einer Umgehungsleitung versehen sein, um bei Außerbetriebsnahme eines Adsorbers den Schritt e) nicht unterbrechen zu müssen.

Vorzugsweise wird der Schritt e) in einer Vorrichtung durchgeführt, in der zumindest zwei parallel geschaltete Adsorbereinheiten vorhanden sind. Diese können gleichzeitig oder bevorzugt wechselweise betrieben werden. Dabei kann jede Adsorbereinheit aus einem oder mehreren Adsorber(n) bestehen. Es können auch mehr als zwei Adsorbereinheiten parallel verschaltet sein. Bei der parallel verschalteten Fahrweise kann in der gerade nicht betriebenen Adsorbereinheit das Adsorptionsmittel ausgetauscht oder regeneriert werden.

Bei der Durchführung des Verfahrensschrittes e) wird ein Adsorber spätestens dann außer Betrieb genommen, wenn die Kapazitätsgrenze für die Aufnahme von Kobaltverbindungen erreicht ist. Ein anderer Grund der Außerbetriebsnahme kann ein Anstieg des Differenzdrucks zwischen Zu- und Ablauf eines Adsorbers sein.

Nach Außerbetriebsnahme eines Adsorbers kann das Adsorptionsmittel im Adsorber regeneriert oder durch neues ersetzt werden. Im erfmdungsgemäßen Verfahren wird das Adsorptionsmittel bevorzugt ausgetauscht. Das ausgetauschte mit Kobaltverbindungen belegte Adsorptionsmittel kann aufgearbeitet oder entsorgt werden.

Durch das erfindungsgemäße Verfahren kann die Standzeit des Hydrierungskatalysators im Schritt d) erhöht werden, da Kobaltverbindungen und auch andere Metallverbindungen, insbesondere die des Eisens und des Nickels, aus dem Hydriereinsatz in Schritt e) entfernt werden können. Weiterhin kann der Aufbau eines Differenzdruckes über die erste Katalysatorschicht verringert werden. Dadurch wird es möglich, die Hydrierung ohne Unterbrechung über eine Revisionsperiode (5 Jahre) zu betreiben.

Im erfindungsgemäßen Verfahren erfolgt die Abtrennung von Spuren von Kobaltverbindungen in Schritt e) durch Adsorption. Je nach Kobaltgehalt in der in Schritt e) zu behandelnden organischen Phase kann die Abtrennung der Kobaltverbindungen allein durch Adsorption erfolgen oder aber alternativ durch eine Kombination von Extraktion (Schritt g)) und Adsorption (Schritt e)) erfolgen. Dabei ist die Extraktion die erste Stufe. Die zweistufige Abtrennung, bei der dem dem Schritt e) zugeführten Gemisch in einem vorangegangenen Schritt g) ein Teil des Kobalts durch ein- oder mehrfache Extraktion mit Wasser entfernt wird, ist die bevorzugte Ausführung der vorliegenden Erfindung. Im erfmdungsgemäßen Verfahren muss nicht jeder einzelnen Extraktionsstufe eine Adsorptionsstufe zugeordnet sein; wichtig ist jedoch, dass der gesamte dem Hydrierreaktor zugeführte organische Strom zumindest eine Adsorptionsstufe (Schritt e)) durchströmt hat.

Die Durchführung des Schrittes g) kann dem Stand der Technik entnommen werden. Die Reduzierung des Restkobaltgehaltes in entkatalysierten Hydroformylierungsgemischen durch Extraktion wird beispielsweise in der Patentanmeldung DE 102 27 995 beschrieben. Die dort beschriebene Extraktion soll als eine mögliche Ausführungsform des Schrittes g) Bestandteil der Offenbarung der vorliegenden Erfindung sein.

In DE 102 27 995 wird durch Extraktion der organischen Phase mit Wasser bevorzugt kontinuierlich im Gegenstrom in üblichen technischen Extraktoren die Konzentration an Kobaltverbindungen (berechnet als metallisches Kobalt) auf vorzugsweise unter 0,5 Massen-ppm gesenkt. Organische Ströme mit solch geringen Kobaltkonzentrationen werden bevorzugt dem Schritt e) zugeführt.

Durch die Anwendung des erfindungsgemäßen Schrittes e), gegebenenfalls mit einer vorherigen Behandlung gemäß Schritt g) kann der Kobaltgehalt in der organischen Phase, die der Hydrierung des Schrittes d) zugeführt werden soll, vorzugsweise auf kleiner 30 Massen-ppb (30*10⁻⁹), bevorzugt kleiner 20 Massen-ppb und besonders bevorzugt kleiner 10 Massen-ppb gesenkt werden. Als Zwischenprodukt des erfindungsgemäßen Verfahrens wird nach der Durchführung des Verfahrensschrittes e) eine zumindest ein Aldehyd mit 7 bis 25 Kohlenstoffatomen aufweisende organische Phase erhalten. Diese aldehydaufweisende organische Phase weist einen wie gerade angegebenen Anteil an Kobalt auf.

Neben dem Adsorptionsschritt e) weist das erfindungsgemäße Verfahren, wie oben beschrieben, die Schritte a) bis d) auf. Diese können wie im Stand der Technik oder nachfolgend beschrieben durchgeführt werden.

Als Edukte für das erfindungsgemäße Verfahren können Olefine oder Gemische von Olefinen mit 6 bis 24 Kohlenstoffatomen, vorzugsweise mit 8 bis 16 Kohlenstoffatomen, bevorzugt mit 8 bis 12 Kohlenstoffatomen oder solche Olefine aufweisende Gemische eingesetzt werden. Die Gemische können Olefine mit end- und/oder innenständige C-C-Doppelbindungen aufweisen. Die Gemische können Olefine gleicher, ähnlicher (± 2) oder deutlich unterschiedlicher (> ± 2) Kohlenstoffatomzahl (C-Zahl) aufweisen oder aus diesen bestehen. Als Olefine, die entweder in reiner Form, in einem Isomerengemisch oder in einem Gemisch mit weiteren Olefinen anderer C-Zahl als Edukt eingesetzt werden können, seien beispielsweise genannt: 1-, 2- oder 3-Hexen, 1-Hepten, lineare Heptene mit innenständiger Doppelbindung (2-Hepten, 3-Hepten usw.), Gemische linearer Heptene, 2- oder 3-Methyl-1-hexen, 1-Octen, lineare Octene mit innenständiger Doppelbindung, Gemische linearer Octene, 2- oder 3-Methylhepten, 1-Nonen, lineare Nonene mit innenständiger Doppelbindung, Gemische linearer Nonene, 2-, 3- oder 4-Methyloctene, 1-, 2-, 3-, 4- oder 5-Decen, 2-Ethyl-1-octen, 1-Dodecen, lineare Dodecene mit innenständiger Doppelbindung, Gemische linearer Dodecene, 1-Tetradecen, lineare Tetradecene mit innenständiger Doppelbindung, Gemische linearer Tetradecene, 1-Hexadecen, lineare Hexadecene mit innenständiger Doppelbindung, Gemische linearer Hexadecene. Geeignete Edukte sind weiterhin u. a. das bei der Dimerisierung von Propen anfallende Gemisch isomerer Hexene (Dipropen), das bei der Dimerisierung von Butenen anfallende Gemisch isomerer Octene (Dibuten), das bei der Trimerisierung von Propen anfallende Gemisch isomerer Nonene (Tripropen), das bei der Tetramerisierung von Propen oder der Trimerisierung von Butenen anfallende Gemisch isomerer Dodecene (Tetrapropen oder Tributen), das bei der Tetramerisierung von Butenen anfallende Hexadecen-Gemisch (Tetrabuten) sowie durch Cooligomerisierung von Olefinen mit unterschiedlicher C-Zahl (bevorzugt 2 bis 4) hergestellte Olefingemische, gegebenenfalls nach destillativer Trennung in Fraktionen mit gleicher oder ähnlicher (±2) C-Zahl. Weiterhin können Olefine oder Olefmgemische, die durch Fischer-Tropsch-Synthese erzeugt worden sind, eingesetzt werden. Darüber hinaus können Olefme, die durch Olefin-Metathese oder durch andere technische Prozesse hergestellt worden sind, verwendet werden. Bevorzugte Edukte sind Gemische isomerer Octene-, Nonene-, Dodecene- oder Hexadecene, d. h. Oligomere von niedrigen Olefmen, wie n-Butenen, Isobuten oder Propen. Andere ebenfalls gut geeignete Edukte sind Oligomere aus C₅-Olefinen, wie z. B. Gemische isomerer Decene.

Für die Oligomerisierung von Butenen zu im Wesentlichen C₈-Olefinen enthaltenden Gemischen gibt es im Prinzip drei Verfahrensvarianten. Lange bekannt ist die Oligomerisierung an sauren Katalysatoren, wobei technisch z. B. Zeolithe oder Phosphorsäure auf Trägern eingesetzt werden. Hierbei werden Isomerengemische von verzweigten Olefinen erhalten, die im Wesentlichen Dimethylhexene darstellen (WO 92/13818). Ein ebenfalls weltweit ausgeübtes Verfahren ist die Oligomerisierung mit löslichen Ni-Komplexen, bekannt als DIMERSOL-Verfahren (siehe J. Schulze, M. Homann, "C4-Hydrocarbons and Derivates", Springer-Verlag, Berlin, Heidelberg 1989, S. 69 und B. CORNILS, W. A. HERRMANN, "Applied Homogeneous Catalysis with Organicmetallic Compounds"; Vol. 1 & 2, VCH, Weinheim, New York 1996). Die dritte Verfahrensvariante ist die Oligomerisierung an Nickel-FestbettKatalysatoren; eines dieser Verfahren ist der OCTOL-Process (Hydrocarbon Process., Int. Ed. (1986) 65 (2. Sect.1) Seiten 31 - 33), bei dem vorzugsweise Nickel-Katalysatoren wie in EP 0 395 857 beschrieben verwendet werden. Für die erfindungsgemäße Herstellung eines C₉₋Alkoholgemischs, das sich insbesondere für die Darstellung von Weichmachern eignet, wird bevorzugt ein C₈-Olefingemisch, das aus linearen Butenen nach dem OCTOL-Prozess gewonnen worden ist, eingesetzt.

### a) Hydroformylierungsreaktion

Die Hydroformylierung der Olefine gemäß Schritt a) erfolgt im erfindungsgemäßen Verfahren in Gegenwart von Kobaltkatalysatoren, bevorzugt unmodifizierte Katalysatoren wie HCo(CO)₄ und/oder Co₂(CO)₈ und von Wasser. Es kann sowohl vorgebildeter Katalysator oder ein Katalysatorvorläufer, wie eine Kobaltverbindung, aus der im Reaktor der eigentliche Katalysator entsteht, in der Hydroformylierungsreaktion eingesetzt werden.

Wenn der fertige, aktive Katalysator (z. B. HCo(CO)₄ und/oder Co₂(CO)₈) eingesetzt wird, wird Wasser, Olefin, Synthesegas und der aktive Katalysator dem Reaktor, in dem die Hydroformylierung durchgeführt wird, zugeführt. Wasser kann dabei schon vor dem Reaktor in das Olefin dispergiert werden, beispielsweise durch Verwendung eines Statikmischers. Es ist jedoch auch möglich, alle Komponenten erst im Reaktor zu vermischen.

Die Menge an Wasser im Hydroformylierungsreaktor kann im weiten Bereich variiert werden. Durch Einstellen des Mengenverhältnis von Wasser und Olefm und der Reaktionsparameter, beispielsweise Temperatur, kann im flüssigen Hydroformylierungsaustrag Wasser homogen gelöst oder zusätzlich dispergiert sein.

Im erfmdungsgemäßen Verfahren wird bevorzugt in Schritt a) der Katalysator (HCo(CO)₄ und/oder Co₂(CO)₈) erst im Hydroformylierungsreaktor erzeugt. Eine solche Verfahrensführung der Hydroformylierung wird beispielsweise in DE 196 54 340 beschrieben. Bei dieser Verfahrensführung werden die Ausgangsstoffe, wie die Kobaltsalzlösung, die organische Phase und das Synthesegas, gleichzeitig, vorzugsweise mit Hilfe einer Mischdüse, im Gleichstrom von unten in den Reaktor eingebracht.

Als Kobaltverbindungen werden bevorzugt Kobaltsalze wie Formiate, Acetate oder Salze von Carbonsäuren, die wasserlöslich sind, verwendet. Besonderes bewährt hat sich Kobaltacetat, das als wässrige Lösung mit einem Kobalt-Gehalt von 0,5 bis 3 Massen-% vorzugsweise von 0,8 bis 1,8 Massen-%, gerechnet als Metall, eingesetzt wird. Ein weitere bevorzugte Einsatzlösung für die Herstellung des Katalysators ist die wässrige Kobaltsalzlösung, die im Trennungsschritt c) anfällt.

Die im Hydroformylierungsreaktor gewünschte Wassermenge kann mit der Kobaltsalzlösung eingebracht werden, deren Konzentration in weitem Bereich variiert werden kann. Es ist jedoch auch möglich, neben der Kobaltsalzlösung zusätzliches Wasser einzuspeisen.

Eine besondere Bedeutung sollte bei dem kobaltkatalysierten Verfahren der Dosierung der Ausgangsstoffe in den Reaktor beigemessen werden. Die Dosiervorrichtung sollte eine gute Phasenvermischung und die Erzeugung einer möglichst hohen Phasenaustauschfläche gewährleisten. Ferner kann es vorteilhaft sein, den Reaktorraum der Hydroformylierungsreaktoren durch den Einbau von 1 bis 10, vorzugsweise 2 bis 4, senkrecht zur Fließrichtung des Reaktanden- und Produktenstromes angeordneten Lochblechen zu unterteilen. Durch die Reaktorkaskadierung wird die Rückvermischung gegenüber der einfachen Blasensäule stark vermindert und das Strömungsverhalten dem eines Rohreaktors angenähert. Diese verfahrenstechnische Maßnahme hat zur Folge, dass sowohl die Ausbeute als auch die Selektivität der Hydroformylierung verbessert werden können.

Weitere bevorzugte Ausführungsformen der Durchführung des Hydroformylierungsschrittes a) können z. B. DE 199 39 491 oder DE 101 35 906 entnommen werden. So wird gemäß DE 199 39 491 aus dem unteren Teil des Reaktors ein Teilstrom der flüssigen Mischphase (wässrige Kobaltsalzlösung/ organische Phase) abgezogen und an einer höheren Stelle des Reaktors wieder eingespeist. Nach DE 101 35 906 wird im Hydroformylierungsreaktor der Stand einer wässrigen Phase konstant gehalten. Die Konzentration an Kobaltverbindungen (berechnet als metallisches Kobalt) in der wässrigen Sumpfphase liegt vorzugsweise im Bereich von 0,4 bis 1,7 Massen-%.

Bevorzugt wird der Verfahrensschritt a) bei einer Temperatur von 100 bis 250 °C, vorzugsweise von 140 bis 210 °C durchgeführt. Der Druck (Synthesegas-Druck), bei dem der Verfahrensschritt a) durchgeführt wird, beträgt vorzugsweise von 10 bis 40 MPa, bevorzugt von 20 bis 30 MPa. Das Volumenverhältnis vom Kohlenmonoxid zum Wasserstoff im Synthesegas beträgt vorzugsweise von 2 : 1 bis 1 : 2, bevorzugt von 1 : 1 bis 1 : 1,5. Das Synthesegas wird vorzugsweise im Überschuss, zum Beispiel bis zu dem Dreifachen der stöchiometrischen Menge, eingesetzt. In dem erfindungsgemäßen Verfahren können ein oder mehrere Verfahrensschritte a) vorhanden sein. Sind mehrere Hydroformylierungsschritte a) im erfindungsgemäßen Verfahren vorhanden, so können gleiche oder unterschiedliche Bedingungen in diesen Verfahrensschritten eingestellt werden. Bei mehrstufigen Verfahrensvarianten wird die Hydroformylierung in der ersten Verfahrensstufe, in der die reaktiveren Olefine umgesetzt werden, bevorzugt bei Temperaturen von 140 bis 195 °C, vorzugsweise von 160 bis 185 °C durchgeführt. In einer ersten von mehreren Verfahrensstufen werden bevorzugt Olefin-Umsätze von 20 bis 95 %, vorzugsweise von 50 bis 80 % angestrebt.

Im flüssigen Hydroformylierungsaustrag beträgt die Konzentration an Kobaltverbindungen (berechnet als metallisches Kobalt) vorzugsweise von 0,01 bis 0,5 Massen-%, bevorzugt von 0,02 bis 0,08 Massen-% (bezogen auf die Summe aus organischer und wässriger Phase).

Durch die unterschiedlichen Möglichkeiten der Wasserzugabe ist der Wassergehalt im Eintrag des Hydroformylierungsreaktors nur schwierig zu bestimmen. Nachfolgend werden daher Angaben über den Austrag des Reaktors gemacht, wobei der Wassergehalt im Reaktoraustrag praktisch gleichbedeutend mit dem Wassergehalt der flüssigen Phase während der Reaktion ist. Die Wasserkonzentrationen in den flüssigen Hydroformylierungsausträgen können von 0,1 bis 10 Massen-%, insbesondere von 0,5 bis 5 Massen-% betragen. Die Wassergehalte der Hydroformylierungsausträge der einzelnen Hydroformylierungsstufen können gleich oder unterschiedlich sein. Bevorzugt ist das Wasser in den flüssigen Hydroformylierungsausträgen homogen gelöst.

Die Reaktoren, in denen die Hydroformylierung des Schrittes a) durchgeführt wird, können, wenn mehrere Verfahrensschritte a) vorhanden sind, in allen Verfahrensschritten gleich oder unterschiedlich sein. Beispiele für einsetzbare Reaktortypen sind Blasensäulenreaktoren, Schlaufenreaktoren, Strahldüsenreaktoren, Rührreaktoren und Rohrreaktoren, die zum Teil kaskadiert und/oder mit Einbauten versehen sein können.

### b) Katalysatorabtrennung

Zur Behandlung eines Hydroformylierungsgemisches, welches z. B. aus einem Schritt a) erhalten wurde, gemäß Schritt b) (Entkobaltung) werden die Produktausträge nach Verlassen der Hydroformylierungsreaktoren vorzugsweise auf 1 bis 3 MPa entspannt und in Gegenwart von sauren, wässrigen Kobalt(II)-salzlösungen ("Prozesswasser") mit sauerstoffhaltigen Gasen, insbesondere Luft oder Sauerstoff umgesetzt und so oxidativ von Kobalt-Carbonylkomplexen befreit. Der Schritt b) wird vorzugsweise bei einer Temperatur von 90 bis 160 °C, bevorzugt von 110 bis 150 °C umgesetzt. Die hydroformylierungsaktiven Kobaltcarbonylkomplexe werden so unter Bildung von Kobalt(II)-salzen zerstört. Die Entkobaltungsverfahren sind gut bekannt und in der Literatur ausführlich, wie z. B von J. FALBE, in "New Syntheses with Carbon Monoxide", Springer Verlag (1980), Berlin, Heidelberg, New York, Seite 158 ff., beschrieben. Die eingesetzten sauren Kobalt(II)-salzlösungen weisen vorzugsweise einen pH-Wert von 1,5 bis 4,5.

Die Behandlung gemäß Schritt b) wird vorzugsweise in einem mit Füllkörpern, wie z. B. Raschig-Ringen, befüllten Druckbehälter, in dem möglichst hohe Phasenaustauschflächen erzeugt werden, durchgeführt.

### c) Trennung des Gemisches aus b)

Ein gemäß Schritt b) erhaltenes Gemisch, welches aus einer an Kobalt abgereicherten organischen, aliphatische Aldehyde enthaltenden Phase und einer Kobalt-reichen wässrigen Phase besteht, wird in Schritt c) vorzugsweise in einem dem Druckbehälter des Schrittes b) nachgeschalteten Trennbehälter in eine wässrige und eine organische Phase getrennt. Die wässrige Phase, das "Prozesswasser", die das zurückextrahierte, aus der organischen Phase wiedergewonnene Kobalt in Form von Kobaltacetat/formiat enthält, kann ganz oder nach Ausschleusung eines geringen Anteils in den Schritt a) zurückgeführt werden. Vorzugsweise wird die wässrige Phase direkt in den Hydroformylierungsreaktor der jeweiligen Verfahrensstufe zurückgeführt und kann als Ausgangsstoff für die in-situ Herstellung der Kobalt-Katalysatorkomplexe verwendet werden.

Optional kann vor der Rückführung des Prozesswassers in den Schritt a) ein Teil der überschüssigen Ameisensäure entfernt werden. Dies kann beispielsweise durch Destillation erfolgen. Eine andere Möglichkeit besteht darin, einen Teil der Ameisensäure zu zersetzen, beispielsweise katalytisch, wie in DE 100 09 207 beschrieben. Weiterhin ist es möglich, aus der bei der Entkobaltung anfallenden Kobaltsalzlösung durch Vorcarbonylierung den eigentlichen Hydroformylierungskatalysator (Co₂(CO)₈ und/oder HCo(CO)₄) herzustellen und diesen in den Schritt a) einzusetzen.

### d) Hydrierung

Die Hydrierung einer aldehydhaltigen Phase gemäß Schritt d) kann in der Gasphase oder in Flüssigphase durchgeführt werden. Bevorzugt wird die Hydrierung gemäß Schritt d) als Flüssigphasenhydrierung durchgeführt. Vorzugsweise wird die Flüssigphasenhydrierung bei einem Gesamtdruck von 0,5 bis 10 MPa, bevorzugt 1,5 bis 5 MPa durchgeführt. Eine Hydrierung in der Gasphase kann auch bei niedrigeren Drücken durchgeführt werden, mit entsprechend großen Gasvolumina. Werden mehrere Hydrierungsreaktoren eingesetzt, können die Gesamtdrücke gleich oder unterschiedlich sein, wobei die Drucke in den einzelnen Reaktoren innerhalb der genannten Druckgrenzen liegen sollten.

Die Temperatur, bei der die Hydrierung gemäß Schritt d) durchgeführt wird, beträgt in flüssiger oder gasförmiger Phase bevorzugt von 120 bis 220 °C, besonders bevorzugt von 140 bis 180 °C. Beispiele für Hydrierungen, die im erfindungsgemäßen Verfahren als Schritt d) eingesetzt werden können, sind z. B. in den Patentanmeldungen DE 198 42 369 und DE 198 42 370 beschrieben.

Im erfindungsgemäßen Verfahren kann die Hydrierung optional in Gegenwart von Wasser durchgeführt werden. Das benötigte Wasser kann im Reaktorzulauf enthalten sein. Es ist jedoch auch möglich, Wasser an geeigneter Stelle in die Hydrierapparatur einzuspeisen. Bei Durchführung des Schrittes d) als Gasphasenhydrierung wird Wasser zweckmäßig in Form von Wasserdampf zugeführt. Ein bevorzugtes Hydrierverfahren, welches als Schritt d) im erfindungsgemäßen Verfahren eingesetzt werden kann, ist die Flüssigphasenhydrierung unter Zusatz von Wasser, wie sie beispielsweise in DE 100 62 448 beschrieben ist. Die Hydrierung wird vorzugsweise bei einem Wassergehalt von 0,05 bis 10 Massen-%, bevorzugt von 0,5 bis 5 Massen-% und besonders bevorzugt von 1 bis 2,5 Massen-% durchgeführt. Der Wassergehalt wird im Hydrieraustrag bestimmt.

Als Katalysatoren können die in den genannten bekannten Verfahren eingesetzten Katalysatoren eingesetzt werden. Bevorzugt werden zur Hydrierung gemäß Schritt b) z. B. Kupfer-, Nickel-, Kupfer/Nickel-, Kupfer/Chrom-, Kupfer/Chrom/Nickel-, Zink/Chrom-, Nickel/Molybdän-Katalysatoren eingesetzt. Die Katalysatoren können trägerfrei sein, oder die hydrieraktiven Stoffe bzw. ihre Vorläufer können auf Trägern, wie beispielsweise Aluminiumoxid, Titandioxid, Zirkondioxid oder Siliziumdioxid, aufgebracht sein.

Bevorzugte Katalysatoren, die in Schritt d) eingesetzt werden, enthalten, jeweils von 0,3 bis 15 Massen-% Kupfer und Nickel sowie als Aktivatoren 0,05 bis 3,5 Massen-% Chrom und vorzugsweise von 0,01 bis 1,6 Massen-%, bevorzugt 0,02 bis 1,2 Massen-% einer Alkalikomponente, wie z. B. Natrium oder Kalium, auf einem Trägermaterial, vorzugsweise Aluminiumoxid und Siliziumdioxid. Die Mengenangaben beziehen sich auf den noch nicht reduzierten Katalysator. Die Alkalikomponente ist optional.

Die Katalysatoren werden vorteilhaft in einer Form eingesetzt, in der sie einen geringen Strömungswiderstand bieten, z. B. in Form von Granalien, Pellets oder Formkörpern, wie Tabletten, Zylindern, Strangextrudaten oder Ringen. Sie werden zweckmäßig vor ihrem Einsatz aktiviert, z. B. durch Erhitzen im Wasserstoffstrom.

### f) Destillative Auftrennung

Es kann vorteilhaft sein, wenn in dem erfindungsgemäßen Verfahren zwischen den Schritten c) und d) ein Schritt f) vorgesehen ist, in dem die in Schritt c) erhaltene organische Phase, z. B. in einer Destillation, in eine Leichtsiederfraktion und eine Aldehyd-haltige Sumpffraktion getrennt wird. Die organische Phase, die nach dem Schritt c) erhalten wird, kann nicht umgesetzte Olefine, Aldehyde, Alkohole, Ameisensäureester und Hochsieder sowie Spuren an Kobaltverbindungen aufweisen. In Schritt f) wird diese Phase in eine Leichtsieder aufweisende Fraktion, die insbesondere die nicht umgesetzten Olefine aufweist und eine Sumpffraktion, welche insbesondere die Wertprodukte (Aldehyde, Alkohole und Formiate) enthält, aufgetrennt. Die Abtrennung der Leichtsieder von den Hydroformylierungsprodukten kann beispielsweise durch Destillation oder Wasserdampfdestillation erfolgen.

Die Leichtsiederfraktion (Kopffraktion) enthält in Schritt a) nicht umgesetzten Olefme und kann durch Hydrierung von Olefmen entstandene Paraffine, gelöstes Wasser und gegebenenfalls geringe Mengen an Wertprodukten aufweisen. Vorzugsweise wird die Leichtsiederfraktion in einen Hydroformylierungsschritt zurückgeführt. Sind mehrere Hydroformylierungsschritte a) in verschiedenen Reaktionsstufen vorhanden, so kann die Leichtsiederfraktion in einen Hydroformylierungsschritt der folgenden Reaktionsstufe geführt oder in den Hydroformylierungsschritt der letzten Reaktionsstufe zurückgeführt werden.

Der optionale Schritt f) kann vor oder nach dem Schritt e) durchgeführt werden. Vorzugsweise wird der Schritt f) vor dem Schritt e) durchgeführt, so dass die organische Phase aus Schritt c) in einem Destillationsschritt f) in eine Leichtsiederfraktion und eine Aldehyd-haltige Sumpffraktion getrennt wird und die aldehydhaltige Sumpffraktion dem Schritt e) zugeführt wird. Auf diese Weise wird die Menge des auf den Adsorber geschickten Materials verringert.

Das erfindungsgemäße Verfahren kann eine oder mehrere Stufen umfassen, die jeweils einen oder mehrere der Schritte a), b), c), d), e) und gegebenenfalls f) aufweisen, wobei in dem erfindungsgemäßen Verfahren in der Summe aller Stufen jeder der Schritte a) bis e) zumindest einmal angewendet bzw. durchlaufen wurde. Die Stufen können insbesondere so verschaltet sein, dass die Schritte d), e) und/oder f) im Gesamtverfahren nur einmal vorhanden sind, während die Schritte a) bis c) in mehreren Stufen, also mehrfach vorhanden sind.

Wird das erfindungsgemäße Verfahren einstufig ausgeführt, so kann die in Schritt c) abgetrennte organische Phase ganz oder teilweise entweder in die Verfahrensstufen e) oder f) geführt werden. Bevorzugt wird die organische Phase nur teilweise weitergeführt, um so einen Auslass für die sich sonst anreichernden aliphatischen Verbindungen zu schaffen.

Es kann vorteilhaft sein, wenn das erfindungsgemäße Verfahren in 2, 3, 4 oder mehr als 4 Stufen durchgeführt wird. Das erfindungsgemäße Verfahren kann bzgl. jeder Verfahrensstufe und jedes Verfahrensschritts kontinuierlich oder diskontinuierlich durchgeführt werden. Bevorzugt werden alle Verfahrensschritte kontinuierlich durchgeführt.

Nachfolgend werden einige Ausführungsformen des erfindungsgemäßen Verfahrens beschrieben, bei denen das Verfahren in mehreren Stufen durchgeführt wird, ohne dass die Erfindung auf diese Ausführungsformen beschränkt sein soll.

### Ausführungsform 1

In dieser Ausführungsform werden mindestens zwei Reaktionsstufen durchlaufen, wobei die in Schritt f) abgetrennte Leichtsiederfraktion in den Schritt a) der folgenden Reaktionsstufe geleitet und die in den Schritten f) aller Reaktionsstufen abgetrennten aldehydhaltigen Sumpffraktionen in einem gemeinsamen Schritt d) hydriert werden. In dieser Verfahrensvariante werden daher die Schritte a), b), c) und f) sukzessive durchlaufen und nur der Schritt d) der Hydrierung der Aldehydfraktion erfolgt gemeinsam für alle Reaktionsstufen.

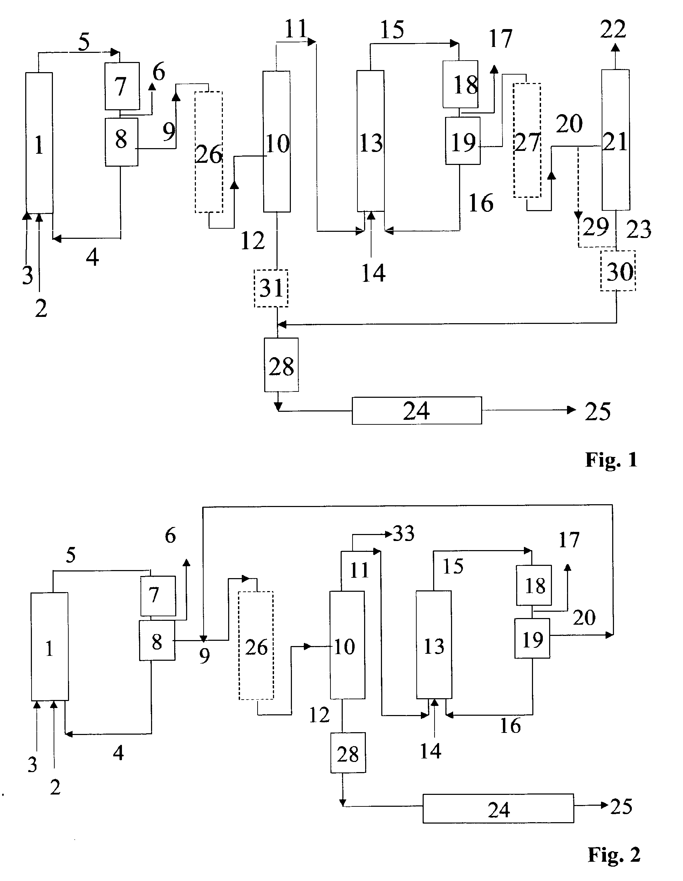

Eine Variante der Ausführungsform 1 des erfmdungsgemäßen Verfahrens wird in Fig. 1 als Blockschema wiedergegeben. Der Schritt a) wird in einem ersten Hydroformylierungsreaktor 1 durchgeführt, in den ein Olefingemisch 3, Synthesegas 2 (Kohlenmonoxid und Wasserstoff) sowie eine wässrige Lösung einer Kobaltverbindung oder Kobaltkatalysator und Wasser 4 eingespeist werden. Das so erhaltene Hydroformylierungsgemisch 5 wird in Schritt b) entspannt und das entspannte Hydroformylierungsgemisch wird in Behälter 7 mit wässriger, saurer Kobalt(II)-salzlösung und Luft behandelt. Das in Behälter 7 erhaltene Gemisch wird in Schritt c) in der ersten Katalysatorabtrennung 8 von Kobaltverbindungen 4 befreit. Das Entspannungsgas 6 (nicht verbrauchtes Synthesegas) wird vor der Katalysatortrennung 8 abgezogen. Die Kobaltverbindungen enthaltende wässrige Phase wird, gegebenenfalls nach Ausschleusung eines kleinen Teilstroms und nach Ergänzung durch frischen Katalysator, in den ersten Hydroformylierungsreaktor 1 zurückgeführt. Unter Katalysator werden hier auch Vorstufen von Katalysatoren, z. B. Kobalt(II)salzlösungen, bezeichnet. Die vom Katalysator befreite organische Phase 9 gelangt von dort in eine Destillationskolonne 10, in der der Trennschritt f) durchgeführt wird und die organische Phase in eine Leichtsiederfraktion 11, die überwiegend aus nicht umgesetzten Olefmen besteht, und Rohaldehyd 12 getrennt wird. Die Leichtsieder 11, Synthesegas 14 und eine wässrige Lösung einer Kobaltverbindung oder ein bereits gebildeter Kobaltkatalysator und Wasser 16 werden in den zweiten Hydroformylierungsreaktor 13 eingebracht (Schritt a) der zweiten Stufe). Das Hydroformylierungsgemisch 15 aus dem zweiten Hydroformylierungsreaktor 13 wird wiederum entspannt und das entspannte Hydroformylierungsgemisch 15 wird nach der zweiten Entkobaltung 18 (Schritt b) der zweiten Stufe) in der zweiten Katalysatorabtrennung 19 vom Katalysator 16 befreit (Schritt c) der zweiten Stufe), der wiederum, gegebenenfalls nach Ausschleusung eines kleinen Teilstroms und nach Ergänzung durch frischen Katalysator, in den zweiten Hydroformylierungsreaktor 13 zurückgeführt wird. Das Entspannungsgas 17 (nicht verbrauchtes Synthesegas) wird vor der Katalysatorabtrennung 19 abgezogen. Das entkatalysierte Hydroformylierungsgemisch 20 wird in der Destillationskolonne 21 (Schritt f) der zweiten Stufe) in eine Leichtsiederfraktion 22, die überwiegend aus gesättigten Kohlenwasserstoffen besteht, und Rohaldehyd 23 aufgetrennt werden. Gegebenenfalls kann ein Teil der Leichtsiederfraktion 22 in den Reaktor 13 zurückgefahren werden. (Leitung in Fig. 1 nicht gezeichnet). Die Sumpffraktionen (Rohaldehyd) aus den Destillationskolonnen 10 und 21 (Schritte f) werden vereinigt und über einen Adsorber 28 (Schritt e) der Hydriereinheit 24 zugeführt, in der Rohaldehyd in Verfahrensschritt d) mit Wasserstoff zum Alkohol 25 hydriert wird, der optional in einer nicht dargestellten Destillation auf reinen Alkohol aufgearbeitet werden kann.

In dieser Ausführungsform der Erfindung weist jede Verfahrensstufe einen Hydroformylierungsschritt a), eine Entkobaltungsschritt b), einen Katalysatorabtrennungsschritt c) und einen Trennschritt f) auf, mit der Maßgabe, dass der in c) abgetrennte Katalysator direkt oder nach Aufarbeitung in den Hydroformylierungsschritt a) der jeweiligen Verfahrensstufe zurückgeführt wird. Optional kann diese Verfahrensvariante auch so durchgeführt werden, dass die zweite Verfahrensstufe keinen Trennschritt f) aufweist und das Hydroformylierungsgemisch 20 über Leitung 29 direkt dem Adsorber 28 zur Durchführung des Verfahrensschrittes e) zugeführt wird.

Die erfindungsgemäße Abtrennung von geringen Mengen an Kobaltverbindungen aus den organischen Phasen bzw. den aldehydhaltigen Fraktionen ist an einer oder mehreren Stellen dieser Verfahrensvariante möglich. So kann der Schritt e) nicht nur an der Stelle durchgeführt werden, an der Adsorber 28 vorgesehen ist, sondern der Schritt e) kann zusätzlich oder an Stelle von Adsorber 28 auch nach den beiden Schritten c) nach der Abtrennung der wässrigen Phase in 8 bzw. 19 in den optionalen Adsorbern 26 und 27 oder nach der Abtrennung der Aldehyd-haltigen Sumpffraktion von der Leichtsiederfraktion in den Destillationskolonnen 10 und 21 in den optionalen Adsorbern 30 und 31 durchgeführt werden. Bevorzugt wird die Abtrennung von geringen Mengen an Kobaltverbindungen gemäß Schritt e) nur einmal, direkt vor der Hydrierstufe, d. h. im Adsorber 28, durchgeführt.

### Ausführungsform 2

In dieser Ausführungsform des erfindungsgemäßen Verfahrens werden zwei Reaktionsstufen durchlaufen, wobei die in Schritt f) der ersten Reaktionsstufe abgetrennten Leichtsieder in den Schritt a) der zweiten Reaktionsstufe und der organische Austrag der Schritte c) beider Stufen in den Schritt f) der ersten Reaktionsstufe geleitet werden. Hier weist jede Reaktionsstufe einen Hydroformylierungsschritt a), einen Entkobaltungsschritt b) und einen Katalysatorabtrennungsschritt c) auf, wobei die abgetrennte Katalysatorphase in den jeweiligen Hydroformylierungsschritt zurückgeleitet wird. Die abgetrennte organische Phase wird in einem für beide Reaktionsstufen gemeinsamen Trennschritt f) in eine Leichtsiederfraktion und eine aldehydhaltigen Subfraktion getrennt. Die so erhaltene Leichtsiederfraktion wird in den Hydroformylierungsschritt a) der zweiten Reaktionsstufe, die abgetrennte Sumpffraktion in den Adsorptionsschritt e) und anschließend den Hydrierschritt d) geleitet.

Ein Blockschema einer Variante dieser Ausführungsform des erfindungsgemäßen Verfahrens ist in Fig. 2 dargestellt. Im Hydroformylierungsreaktor 1 der ersten Verfahrensstufe werden ein Olefmgemisch 3, Synthesegas 2 (Kohlenmonoxid und Wasserstoff) sowie eine wässrige Lösung einer Kobaltverbindung oder Kobaltkatalysator zusammen mit Wasser eingespeist. Das in Schritt a) der ersten Stufe erhaltene Hydroformylierungsgemisch 5 wird entspannt, und das entspannte Hydroformylierungsgemisch nach der mit wässriger, saurer Kobalt(II)-salzlösung und Luft durchgeführten Behandlung gemäß Schritt b) (Entkobaltung) 7 in der ersten Katalysatorabtrennung 8 gemäß Schritt c) von Kobaltverbindungen 4 befreit. Das Entspannungsgas 6 (nicht verbrauchtes Synthesegas) wird vor der Katalysatorabtrennung 8 abgezogen. Die Kobaltsalze enthaltende wässrige Phase wird, gegebenenfalls nach Ausschleusung eines kleinen Teilstroms und nach Ergänzung durch frischen Katalysator, in den ersten Hydroformylierungsreaktor 1 zurückgeführt. Die in der Abtrennung gemäß Schritt c) erhaltene entkobaltete organische Phase 9 wird in die Destillationskolonne 10 (Schritt f)) geleitet. Dort wird sie zusammen mit dem entkobalteten Hydroformylierungsgemisch 20 aus dem zweiten Hydroformylierungsreaktor 13 in eine Fraktion 11, die die nicht umgesetzten Olefine und inerte Paraffine enthält, und Rohaldehyd 12 getrennt. Die Leichtsiederfraktion 11 wird, nach Ausschleusung eines Teilstroms 33 zur Abtrennung von gesättigten Kohlenwasserstoffen (Paraffine) und sonstigen, nicht olefinischen Verbindungen, zusammen mit Synthesegas 14 sowie einer wässrigen Lösung einer Kobaltverbindung oder einem Gemisch 16 aus Kobaltkatalysator und Wasser in den zweiten Hydroformylierungsreaktor 13 zur Durchführung des Schrittes a) der zweiten Stufe geführt. Das so erhaltene Hydroformylierungsgemisch 15 wird entspannt und das entspannte Hydroformylierungsgemisch nach einer Entkobaltung 18 gemäß Schritt b) in der zweiten Katalysatorabtrennung 19 gemäß Schritt c) vom Katalysator 16 befreit, der, gegebenenfalls nach Ausschleusung eines kleinen Teilstroms und nach Ergänzung durch frischen Katalysator, in den zweiten Hydroformylierungsreaktor 13 zurückgeführt wird. Das Entspannungsgas 17 (nicht verbrauchtes Synthesegas) wird vor der Katalysatorabtrennung 19 abgezogen Das entkobaltete zweite Hydroformylierungsgemisch 20 wird mit dem Hydroformylierungsgemisch 9 der ersten Stufe, wie bereits erwähnt, in die Trennstufe 10 eingespeist. Der als Sumpffraktion in der Destillationskolonne des Schrittes f) anfallende Rohaldehyd 12 wird im Absorber 28 gemäß Schritt e) von restlichem Kobalt befreit und anschließend in der Hydriereinheit 24 mit Wasserstoff zum Rohalkohol 25 hydriert. Dieser Alkohol kann in einer nicht dargestellten Destillation auf den reinen Alkohol aufgearbeitet werden.

Anstelle oder zusätzlich zur Durchführung des Schrittes e) im Adsorber 28 kann der Schritt e) auch nach der Zusammenführung der organischen Phasen 9 und 32 aus den Abtrennschritten c) der ersten und zweiten Stufe vor der Zuführung in die Destillationskolonne 10 des Schrittes f) in Adsorber 26 erfolgen.

Die Ausschleusung der gesättigten Kohlenwasserstoffe kann anstatt über den Teilstrom 33 auch durch Aufarbeitung eines Teilstroms des entkobalteten Hydroformylierungsprodukts 20 erfolgen (nicht dargestellt). Technisch ist dies zum Beispiel durch eine destillative Auftrennung dieses Teilstroms in Leichtsieder, die ausgeschleust werden, und Aldehyde, die in das entkobaltete Hydroformylierungsgemisch 20 oder den Rohaldehyd 12 zurückgeführt werden, realisierbar.

Diese Ausführungsform des erfindungsgemäßen Verfahrens weist für jede Verfahrensstufe einen Hydroformylierungsschritt a), einen Entkobaltungsschritt b) sowie einen Katalysatorabtrennungsschritt c) auf, wobei die vereinigten flüssigen Hydroformylierungsgemische, in einem gemeinsamen Destillationsschritt f) in Leichtsieder- und Sumpffraktion getrennt werden. Der in den Schritten c) abgetrennte Katalysator kann direkt oder nach Aufarbeitung in den Hydroformylierungsschritt a) der jeweiligen Verfahrensstufe zurückgeführt werden.

### Ausführungsform 3

In dieser Ausführungsform des erfindungsgemäßen Verfahrens werden zwei Reaktionsstufen durchlaufen, wobei die in Schritt f) der ersten Reaktionsstufe abgetrennten Leichtsieder in den Schritt a) der zweiten Reaktionsstufe geleitet und die Schritte b), c) und d) für beide Reaktionsstufen gemeinsam durchgeführt werden.

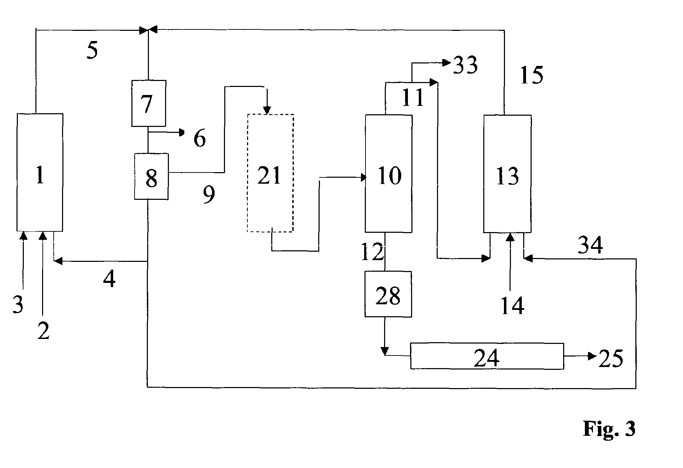

Eine Variante dieser Ausführungsform des erfindungsgemäßen Verfahrens ist als Blockschema in Fig. 3 dargestellt. In den ersten Hydroformylierungsreaktor 1 werden ein Olefmgemisch 3, Synthesegas 2 (Kohlenmonoxid und Wasserstoff) sowie eine wässrige Lösung einer Kobaltverbindung oder ein Gemisch aus Kobaltkatalysator und Teilstrom 4 eingespeist. Das so in Schritt a) der ersten Reaktionsstufe erhaltene Hydroformylierungsgemisch 5 wird zusammen mit dem Hydroformylierungsgemisch 15 aus dem zweiten Hydroformylierungsreaktor 13 (Schritt a) der zweiten Verfahrensstufe) als vereinigte Hydroformylierungsausträge entspannt und nach der Entkobaltung 7 (Schritt b)) wird die organische Phase in der Katalysatorabtrennung 8 gemäß Schritt c) vom Katalysator befreit. Das nicht verbrauchte Synthesegas 6 wird vor der Katalysatorabtrennung 8 abgezogen. Man erhält ein Gemisch 9, das die gebildeten Aldehyde, Alkohole und nicht umgesetzte Olefine enthält. Der Katalysator wird, gegebenenfalls nach Ausschleusung einer Teilmenge und Ergänzung durch frischen Katalysator, in die beiden Teilströme 4 und 34 aufgeteilt. Teilstrom 4 wird in den Hydroformylierungsreaktor 1 der ersten Verfahrensstufe und Teilstrom 34 in den Hydroformylierungsreaktor 13 der zweiten Verfahrensstufe zurückgefahren. Der entkobaltete Hydroformylierungsaustrag 9 wird in der Destillationskolonne 10 des Verfahrensschrittes a) in die Leichtsiederfraktion 11 und den Rohaldehyd (Sumpffraktion) 12 aufgetrennt. Die Leichtsiederfraktion 11, die die nicht umgesetzten Olefme enthält, wird, gegebenenfalls nach Ausschleusung einer Teilmenge 33 zur Abtrennung von gesättigten Kohlenwasserstoffen oder sonstigen nicht olefinischen Verbindungen, zusammen mit Synthesegas 14 und wässriger Lösung einer Kobaltverbindung oder einem Gemisch aus Kobaltkatalysator und Wasser 34 in den zweiten Hydroformylierungsreaktor 13 (Schritt a) der zweiten Verfahrensstufe) eingeleitet. Der Rohaldehyd 12 wird im Absorber 28 in Schritt e) von Kobalt weitestgehend befreit und kann anschließend in der Hydriereinheit 24 mit Wasserstoff zum Rohalkohol 25 hydriert werden. Dieser kann wiederum in einer nicht dargestellten Destillation auf reinen Alkohol aufgearbeitet werden.

Anstelle oder zusätzlich zur Durchführung des Schrittes e) im Adsorber 28 kann der Schritt e) auch vor der Zuführung der organischen Phasen 9 in die Destillationskolonne 10 des Schrittes f) in Adsorber 21 durchgeführt werden.

Auch bei dieser Ausführungsform des erfindungsgemäßen Verfahrens ist es möglich, die Ausschleusung von gesättigten Kohlenwasserstoffen über eine separate Aufarbeitung eines Teilstroms des Hydroformylierungsgemisches 15 durchzuführen, zum Beispiel durch destillative Abtrennung der Leichtsieder.

Diese dritte Ausführungsform des erfindungsgemäßen Verfahrens ist dadurch gekennzeichnet, dass die vereinigten Reaktorausträge aller Hydroformylierungsschritte a) nur eine Entkobaltung b) und einen Katalysatorabtrennungsschritt c) und einen Olefin-Abtrennungsschritt f) durchlaufen. Der im Verfahrensschritt c) abgetrennte Katalysator wird direkt oder nach einer Aufarbeitung aufgeteilt und in die Hydroformylierungsschritte a) der einzelnen Verfahrensstufen zurückgeführt.

Die folgenden Beispiele sollen die Erfindung erläutern, ohne ihre Anwendungsbreite einzuschränken, die sich aus den Patentansprüchen und der Beschreibung ergibt.

### Beispiel 1

In einen Adsorber (Rohr mit einem inneren Durchmesser von 50 mm) wurden 2 l Hydrierkontakt H 14154 (Lieferfirma Degussa AG, Düsseldorf) (1400 g) gefüllt, das als zylindrisches Extrudat mit einem Durchmesser von 1,2 mm und einer Länge von 2 bis 10 mm vorlag. Die Länge der Absorberschicht betrug somit 102 cm. Durch den senkrecht stehenden Absorber wurde ein Hydroformylierungsgemisch aus einer Isononanal-Anlage mit einem Gehalt an Kobaltverbindungen (als Kobalt ausgewiesen) von 75 Massen-ppb (75*10⁻⁹ g/g) mit einer Leerrohrgeschwindigkeit von 61 m/h, was einer Volumengeschwindigkeit im Leerrohr von ca. 110 l/h entspricht, gepumpt. Der Absorber wurde bei einer Temperatur von 70 °C und bei 3,1 MPa absolut betrieben. Der Adsorber wurde von oben nach unten durchströmt.

Nachdem 283 m³ Hydroformylierungsgemisch durch das Adsorptionsmittel geströmt waren, wurde der Adsorber geöffnet und das gesamte Adsorptionsmittel ohne Quervermischung in acht Säulenabschnitten entnommen. Jedem Säulenabschnitt konnte die Lage innerhalb der Adsorptionssäule und eine mittlere Bettlage, Entfernung von der Oberfläche der Anströmseite, zugeordnet werden. Jede der acht Proben wurde getrocknet, homogenisiert und anschließend in ihnen spektroskopisch (AAS) die Kobaltkonzentrationen bestimmt und danach die absolute Menge an Kobalt in dem jeweiligen Säulenabschnitt und die durchschnittliche spezifische Beladung (Gramm Kobalt bezogen auf ein Gramm des eingesetzten Adsorptionsmittel) berechnet. In der nachfolgenden Tabelle 1 wurden diese Werte zusammengestellt.

**Tabelle 1: Ergebnisse der Auswertung von Beispiel 1**

| Nummer des Säulenabschnitts | Bettabschnitt in cm | Mittlere Bettlage in cm | Spezifische Kobaltbeladung in g/g | Absolute Kobaltmenge in g |
|---|---|---|---|---|
| 1 | 0 - 2 | 1 | 0,0185 | 0,508 |
| 2 | 2 - 4 | 3 | 0,0199 | 0,545 |
| 3 | 4 - 8 | 6 | 0,0157 | 0,863 |
| 4 | 8 - 24 | 16 | 0,0193 | 4,236 |
| 5 | 24 - 42 | 33 | 0,0135 | 3,345 |
| 6 | 42 - 62 | 52 | 0,0110 | 3,032 |
| 7 | 62 - 93 | 77,5 | 0,0050 | 2,122 |
| 8 | 93 - 102 | 97,5 | 0,0047 | 0,581 |
| | | | Summenwert | 15,322 |

Während des Versuches wurden mit dem Hydroformylierungsgemisch Kobaltverbindungen mit insgesamt 17,025 g Kobalt in den Absorber geleitet. Davon wurden 15,322 g adsorbiert. Dies entspricht eine Rückhaltequote von 90 %. Der Gehalt an Kobalt im Raffinat betrug nur noch 7,5 Massen-ppb. Damit zeigte der Versuch, dass durch Adsorption unter technischen Bedingungen auch im Spurenbereich der Kobaltgehalt mit geringem Aufwand deutlich gesenkt werden kann. Ein ähnlich gutes Ergebnis wird bei Einsatz eines Hydrierkontaktes erhalten, der als Hydrierkontakt wegen mangelnder Aktivität nicht mehr geeignet ist.

### Beispiel 2

Es wurde die gleiche Apparatur wie in Beispiel 1 verwendet. Sie war mit 2 1 Aktiv-Kohle der Firma Chemviron gefüllt. Zur Adsorption strömte das gleiche Edukt mit der gleichen Raumgeschwindigkeit und bei gleicher Temperatur durch das Adsorptionsrohr. Nach dem Durchsatz von 647 m³ (519 t) Hydroformylierungsgemisch wurde die Kobaltmenge auf dem gesamten Adsorptionsmittel bestimmt. Diese betrug 3,3 g.

### Beispiel 3

Beispiel 3 wurde analog Beispiel 2 durchgeführt. Als Adsorptionsmittel wurde Siliziumdioxid KC-Siliperl AF 125 der Kalichemie eingesetzt. Nach Durchsatz von 1060 m³ (850 t) Hydroformylierungsgemisch waren 1,6 g Kobalt adsorbiert.

Die drei Beispiele zeigen, dass in Hydroformylierungsgemischen, die Kobaltverbindungen im Spurenbereich (ppb-Konzentrationsbereich) enthalten, durch Adsorption der Kobaltgehalt gesenkt werden kann. Ein besonders gut geeignetes Adsorptionsmittel dafür ist der Hydrierkontakt H 14154, der einen Al₂O₃-Träger aufweist.

## Patentansprüche

1. Verfahren zur Herstellung von aliphatischen Alkoholen mit 7 bis 25 Kohlenstoffatomen, bei dem zumindest einmal die Verfahrensschritte
a) Kobalt-katalysierte Hydroformylierung von Olefinen mit 6 bis 24 Kohlenstoffatomen,
b) Behandlung eines Hydroformylierungsgemisches mit Sauerstoff-haltigen Gasen in Gegenwart von sauren, wässrigen Kobalt(II)salzlösungen,
c) Trennung eines gemäß Schritt b) erhaltenen Gemisches in eine Kobaltsalze enthaltende wässrige und eine die aliphatischen Aldehyde enthaltende organische Phase und
d) Hydrierung einer Aldehyd-haltigen organischen Phase durchgeführt werden,
**dadurch gekennzeichnet,**
**dass** die Aldehyd-haltige organische Phase vor der Hydrierung d) zur Abtrennung von Kobaltverbindungen zumindest teilweise in einem Schritt e) mit einem Adsorptionsmittel behandelt wird.

2. Verfahren nach Anspruch 1,
**dadurch gekennzeichnet,**
**dass** als Adsorptionsmittel Aluminiumoxid (Al₂O₃), Siliziumoxid (SiO₂), Alumosilikate oder Aktivkohle oder ein zumindest einen dieser Stoffe aufweisendes Material eingesetzt wird.

3. Verfahren nach Anspruch 1 oder 2,
**dadurch gekennzeichnet,**
**dass** als Adsorptionsmittel gebrauchte oder neue (Hydrier-)Katalysatoren eingesetzt werden.

4. Verfahren nach Anspruch 3,
**dadurch gekennzeichnet,**
**dass** Hydrierkatalysatoren eingesetzt werden, die Aluminiumoxid (Al₂O₃), Siliziumoxid (SiO₂), Alumosilikate oder Aktivkohle als Trägermaterialien aufweisen.

5. Verfahren nach einem der Ansprüche 1 bis 4,
**dadurch gekennzeichnet,**
**dass** der Schritt e) bei einer Temperatur von 5 bis 250 °C durchgeführt wird.

6. Verfahren nach einem der Ansprüche 1 bis 5,
**dadurch gekennzeichnet,**
**dass** der Schritt e) bei einem Druck von 0,1 bis 20 MPa durchgeführt wird.

7. Verfahren nach einem der Ansprüche 1 bis 6,
**dadurch gekennzeichnet,**
**dass** der Schritt e) ohne Zusatz oder mit Zusatz von Wasserstoff durchgeführt wird.

8. Verfahren nach einem der Ansprüche 1 bis 7,
**dadurch gekennzeichnet,**
**dass** der Schritt e) innerhalb oder außerhalb eines Reaktors durchgeführt wird, in dem eine Hydrierung gemäß Schritt d) durchgeführt wird.

9. Verfahren nach einem der Ansprüche 1 bis 7,
**dadurch gekennzeichnet,**
**dass** der Schritt e) in einem Adsorber oder mehreren Adsorbern durchgeführt wird.

10. Verfahren nach Anspruch 9,
**dadurch gekennzeichnet,**
**dass** der Schritt e) in zwei parallel zueinandergeschalteten Adsorbereinheiten durchgeführt wird, wobei jede Adsorbereinheit aus einem oder mehreren Adsorbern bestehen kann.

11. Verfahren nach Anspruch 9,
**dadurch gekennzeichnet,**
**dass** der Schritt e) in einer Vorrichtung durchgeführt wird, in der zumindest zwei parallel geschaltete Adsorbereinheiten vorhanden sind und die beiden Adsorbereinheiten wechselweise betrieben werden.

12. Verfahren nach Anspruch 11,
**dadurch gekennzeichnet,**
**dass** in der gerade nicht betriebenen Adsorbereinheit das Adsorptionsmittel ausgetauscht oder regeneriert wird.

13. Verfahren nach einem der Ansprüche 1 bis 12,
**dadurch gekennzeichnet,**
**dass** dem dem Schritt e) zugeführten Gemisch in einem vorangegangenen Schritt g) ein Teil des Kobalts durch ein- oder mehrfache Extraktion mit Wasser entfernt wird.

14. Verfahren nach einem der Ansprüche 1 bis 13,
**dadurch gekennzeichnet,**
**dass** die organische Phase aus c) in einem Destillationsschritt f) in eine Leichtsiederfraktion und eine aldehydhaltige Sumpffraktion getrennt wird und die aldehydhaltige Sumpffraktion dem Schritt e) zugeführt wird.

## Claims

1. Process for preparing aliphatic alcohols having from 7 to 25 carbon atoms, in which the process steps
a) cobalt-catalyzed hydroformylation of olefins having from 6 to 24 carbon atoms,
b) treatment of a hydroformylation mixture with oxygen-containing gases in the presence of acidic, aqueous cobalt(II) salt solutions
c) separation of a mixture obtained according to step b) into an aqueous phase comprising cobalt salts and an organic phase comprising the aliphatic aldehydes and
d) hydrogenation of an aldehyde-containing organic phase
are carried out at least once, **characterized in that** at least part of the aldehyde-containing organic phase is treated with an adsorbent in a step e) to separate off cobalt compounds prior to the hydrogenation d).

2. Process according to Claim 1, **characterized in that** the adsorbent used is aluminum oxide (Al₂O₃), silicon oxide (SiO₂), aluminosilicates or activated carbon or a material comprising at least one of these substances.

3. Process according to Claim 1 or 2, **characterized in that** the adsorbent used is an exhausted or fresh (hydrogenation) catalyst.

4. Process according to Claim 3, **characterized in that** a hydrogenation catalyst comprising aluminum oxide (Al₂O₃), silicon oxide (SiO₂), aluminosilicates or activated carbon as support material is used.

5. Process according to any of Claims 1 to 4, **characterized in that** step e) is carried out at a temperature of from 5 to 250°C.

6. Process according to any of Claims 1 to 5, **characterized in that** step e) is carried out at a pressure of from 0.1 to 20 MPa.

7. Process according to any of Claims 1 to 6, **characterized in that** step e) is carried out with or without addition of hydrogen.

8. Process according to any of Claims 1 to 7, **characterized in that** step e) is carried out within or outside a reactor in which a hydrogenation according to step d) is carried out.

9. Process according to any of Claims 1 to 7, **characterized in that** step e) is carried out in an adsorber or a plurality of adsorbers.

10. Process according to Claim 9, **characterized in that** step e) is carried out in two adsorber units connected in parallel, with each adsorber unit being able to comprise one or more adsorbers.

11. Process according to Claim 9, **characterized in that** step e) is carried out in an apparatus in which at least two adsorber units connected in parallel are present and the two adsorber units are operated alternately.

12. Process according to Claim 11, **characterized in that** the adsorbent in the adsorber unit which is not currently in operation is replaced or regenerated.

13. Process according to any of Claims 1 to 12, **characterized in that** part of the cobalt in the mixture fed to step e) is removed by single-stage or multiple extraction with water in a preceding step g).

14. Process according to any of Claims 1 to 13, **characterized in that** the organic phase from c) is separated into a low-boiling fraction and an aldehyde-containing bottom fraction in a distillation step f) and the aldehyde-containing bottom fraction is passed to step e).

## Revendications

1. Procédé en vue de la fabrication d'alcools aliphatiques ayant de 7 à 25 atomes de carbone, lors duquel on effectue au moins une fois les étapes de procédé
a) d'hydroformylation, catalysée au cobalt, d'oléfines ayant de 6 à 24 atomes de carbone,
b) de traitement d'un mélange d'hydroformylation à l'aide de gaz contenant de l'oxygène en présence de solutions aqueuses acides de sels de cobalt (II),
c) de séparation d'un mélange obtenu conformément à l'étape b) dans une phase aqueuse contenant les sels de cobalt et dans une phase organique contenant des aldéhydes aliphatiques et
d) d'hydrogénation d'une phase organique contenant des aldéhydes, **caractérisé en ce que** la phase organique contenant des aldéhydes est traitée avant l'hydrogénation d), en vue de la séparation des composés du cobalt, tout au moins partiellement dans une étape e) à l'aide d'un agent d'adsorption.

2. Procédé selon la revendication 1, **caractérisé en ce que** l'on utilise, en tant qu'agent d'adsorption, l'oxyde d'aluminium (Al₂O₃), l'oxyde de silicium (SiO₂), les aluminosilicates ou le charbon actif ou un matériau présentant au moins une de ces substances.

3. Procédé selon la revendication 1 ou 2, **caractérisé en ce que** l'on utilise, en tant qu'agent d'adsorption, des catalyseurs (d'hydrogénation) usagés ou neufs.

4. Procédé selon la revendication 3, **caractérisé en ce que** l'on utilise des catalyseurs d'hydrogénation, qui présentent, en tant que matériaux de support, de l'oxyde d'aluminium (Al₂O₃), de l'oxyde de silicium (SiO₂), des aluminosilicates ou du charbon actif.

5. Procédé selon l'une quelconque des revendications 1 à 4, **caractérisé en ce que** l'étape e) est effectuée à une température de 5 à 250 °C.

6. Procédé selon l'une quelconque des revendications 1 à 5, **caractérisé en ce que** l'étape e) est effectuée à une pression de 0,1 à 20 MPa.

7. Procédé selon l'une quelconque des revendications 1 à 6, **caractérisé en ce que** l'étape e) est effectuée sans addition ou avec addition d'hydrogène.

8. Procédé selon l'une quelconque des revendications 1 à 7, **caractérisé en ce que** l'étape e) est effectuée au sein ou à l'extérieur d'un réacteur, dans lequel on effectue une hydrogénation selon l'étape d).

9. Procédé selon l'une quelconque des revendications 1 à 7, **caractérisé en ce que** l'étape e) est effectuée dans un adsorbeur ou dans plusieurs adsorbeurs.

10. Procédé selon la revendication 9, **caractérisé en ce que** l'étape e) est effectuée dans deux unités d'adsorbeurs branchées en parallèle l'une par rapport à l'autre, chaque unité d'adsorbeur pouvant se composer d'un ou de plusieurs adsorbeurs.

11. Procédé selon la revendication 9, **caractérisé en ce que** l'étape e) est effectuée dans un dispositif, dans lequel sont présentes au moins deux unités d'adsorbeurs branchées en parallèle et dans lequel les deux unités d'adsorbeurs peuvent être opérées en alternance.

12. Procédé selon la revendication 11, **caractérisé en ce que**, dans l'unité d'adsorbeur qui est justement hors fonctionnement, l'on échange ou l'on régénère l'agent d'adsorption.

13. Procédé selon l'une quelconque des revendications 1 à 12, **caractérisé en ce que** l'on élimine, du mélange acheminé à l'étape e) dans une étape antérieure g), une partie du cobalt grâce à une extraction à une reprise ou à plusieurs reprises à l'aide d'eau.

14. Procédé selon l'une quelconque des revendications 1 à 13, **caractérisé en ce que** la phase organique provenant de c) est séparée dans une étape de distillation f) en une fraction de susbtances à point d'ébullition faible et en une fraction de puits contenant des aldéhydes et **en ce que** la fraction de puits contenant des aldéhydes est acheminée à l'étape e).
